# EUROPEAN PATENT APPLICATION

(11) **EP 4 643 894 A1**
(43) Date of publication of application: **05.11.2025**
(21) Application number: 23912214.6
(22) Date of filing: 27.12.2023
(51) Int. Cl.: A61L 27/38, A61B 3/10, A61K 35/30, A61P 27/02, A61P 41/00, C12N 5/071, C12N 5/0789, G01N 21/17

(54) **METHOD FOR MANUFACTURING RETINAL SHEET**

(30) Priority: 28.12.2022 JP 2022210998
(71) Applicant: VCCT Inc., Hyogo 650-0047 (JP); Kobe City Hospital Organization, Kobe-shi, Hyogo 6500047 (JP)
(72) Inventor: MANDAI Michiko, Kobe-shi, Hyogo 650-0047 (JP); MASUDA Tomohiro, Kobe-shi, Hyogo 650-0047 (JP); NISHIDA Mitsuhiro, Kobe-shi, Hyogo 650-0047 (JP)
(74) Representative: Jones, Nicholas Andrew
(86) International application number: PCT/JP2023/046832
(87) International publication number: WO 2024/143436

(57) **Abstract**

The present invention relates to a method for producing a sheet for transplantation from a retinal organoid derived from pluripotent stem cells, and the like. Specifically, the present invention relates to a method for producing a retinal sheet for transplantation from a retinal organoid derived from pluripotent stem cells, the method including a step of performing optical coherence tomography (OCT) on the retinal organoid derived from pluripotent stem cells to identify, as a region suitable for transplantation, a region with a thickness of 70 µm or more and free of foreign structures characterized by differences in brightness, and a step of excising a fragment from the region suitable for transplantation to obtain the retinal sheet for transplantation.

## Description

### Technical Field

### Related Applications:

The present application claims priority based on Patent Application No. 2022-210998 (filed on December 28, 2022), the contents of which are incorporated herein by reference.

### Technical Field:

The present invention relates to a method for producing a sheet for transplantation from a retinal organoid derived from pluripotent stem cells.

### Background Art

In hereditary retinal degenerative diseases, the area of photoreceptor cell degeneration is often extensive; however, the location and size of the degenerative area vary with each disease and stage of degeneration. For photoreceptor degenerative diseases, the potential to restore visual function has been demonstrated through transplantation of retinal cells derived from fetal retina or ES/iPS cells, or sheets excised from retinal organoids. As the transplantation method, a method of transplanting purified photoreceptor cells as a cell suspension and a method of transplanting retinal organoid tissue have been reported. However, in the case of cell transplantation, it remains difficult to efficiently purify the target cells, and there are issues such as the labor and cost associated with purification, the instability of the graft, and the like.

Since grafts excised from retinal organoids have a major axis of about 1 mm, a plurality of grafts must be inserted to cover the area where visual function is desired. However, due to the current manufacturing processes and quality control methods, it is extremely difficult to prepare an adequate quantity. On the other hand, even if a large retinal sheet can be prepared, in retinas with advanced degeneration, transplantation through a large incision may cause damage during transplantation.

Known methods for evaluating the quality of grafts excised from retinal organoids includes a method in which quantitative PCR is performed on an organoid of about 1-2 mm in size at a portion (ring) considered to be equivalent to the product (cap), to confirm the expression of target retinal marker genes and that the expression levels of non-retinal markers or anterior segment marker genes are below predefined threshold values (Patent Literature 1). However, since retinal organoids themselves are 1-2 mm in size with individual variability, inspection of all products is inefficient and associated with high cost. Another problem is that the excision of the cap-ring requires the visual assessment and technical skill of an expert, and thus can only be performed by some technical experts.

### Citation List

### Patent Literature

Patent Literature 1: WO2020/184720

### Summary of Invention

### Technical Problem

The object of the present invention is primarily to provide a method for efficiently producing a retinal sheet with a quality suitable for transplantation, without relying on the advanced discriminatory skills of an expert.

### Solution to Problem

The present inventors have found that it is possible to readily prepare a transplantation sheet with predetermined quality by using optical coherence tomography to identify areas suitable for the transplantation sheet from a retinal organoid produced using a planar organoid differentiation method.

The present invention is based on the above findings, and provides the following [1] to [13]:
[1] A method for producing a retinal sheet for transplantation from a retinal organoid derived from pluripotent stem cells, the method comprising: a step of performing one or more selected from optical coherence tomography (OCT), confocal image analysis, and acoustic microscopy analysis (preferably OCT) on the retinal organoid derived from pluripotent stem cells to identify, as a region suitable for transplantation, a region with a thickness of 70 µm or more (preferably 70 to 175 µm, optionally 80 µm or more, 80 to 150 µm) and free of foreign structures characterized by differences in brightness, and a step of excising a fragment from the region suitable for transplantation to obtain the retinal sheet for transplantation.
[2] The method according to [1], wherein the region free of foreign structures is identified by sequentially extracting a plurality of horizontal X-Y planar images reconstructed from OCT cross-sectional images, along the Z direction, and evaluating them.
[3] The method according to [1] or [2], wherein the region with a thickness of 70 µm or more (preferably 70 to 175 µm, optionally 80 µm or more, 80 to 150 µm) is identified using a heat map.
[4] The method according to any one of [1] to [3], wherein the excised fragment is of a size that can be loaded into an 18G to 24G cannula.
[5] The method according to any one of [1] to [4], wherein the excised fragment has a length of 1.0 to 10.0 mm in the long axis direction and a length of 0.5 to 2.0 mm in the short axis direction.
[6] The method according to any one of [1] to [5], further comprising a step of performing bright-field microscopy and/or phase-contrast microscopy on the retinal organoid derived from pluripotent stem cells, wherein the identification of the region suitable for transplantation by the OCT is performed on the region where retinal differentiation and homogeneity can be confirmed by the bright-field microscopy and/or phase-contrast microscopy.
[7] The method according to any one of [1] to [6], wherein the retinal organoid is a planar retinal organoid with a diameter of 1 cm or more.
[8] A method for producing a composite sheet for retinal transplantation, the method comprising: a step of producing a retinal sheet for transplantation by the method according to any one of [1] to [7]; and a step of laminating the retinal sheet for transplantation onto a retinal pigment epithelial cell sheet via a flowable gel (e.g., a gel containing one or more selected from hyaluronic acid, chondroitin sulfate, and derivatives thereof) to produce the composite sheet for retinal transplantation.
[9] A medicament for the treatment of retinal degenerative diseases, comprising a plurality of cell sheet for retinal transplantations excised from a single planar retinal organoid derived from pluripotent stem cells, and a transplantation medium, wherein the plurality of cell sheet for retinal transplantations each have a size with a length of 1.0 to 10.0 mm in the long axis direction and 0.5 to 2.0 mm in the short axis direction, and have been confirmed by optical coherence tomography (OCT) to have a thickness of 70 µm or more (preferably 70 to 175 µm, optionally 80 µm or more, 80 to 150 µm) and to be free of foreign structures.
[10] The method according to any one of [1] to [8], further comprising a step of performing quantitative PCR on the excised fragment to confirm that the expression levels of markers for extraretinal cells are low and that the expression levels of markers for retinal cells are high. The confirmation step can be carried out, for example, by analyzing absolute or relative expression patterns or expression ratios using a radar chart or the like.
[11] The method according to [10], wherein the marker for non-transplant tissue-derived cells comprises one or more selected from CRYAB, EMX2, s100A10, Zic1, AQP1, and MSX1, preferably one or more selected from CRYAB, Zic1, and EMX2, and the marker for retinal cells comprises one or more selected from Crx, Recoverin, and Rxr-gamma.
[12] A method for treating retinal degenerative diseases, comprising producing a plurality of retinal sheets for transplantation from a retinal organoid derived from pluripotent stem cells by the method according to any one of [1] to [7], [10], and [11], and transplanting the plurality of retinal sheets for transplantation into the retina of a subject in need thereof.
[13] A method for treating retinal degenerative diseases, comprising producing a composite sheet for retinal transplantation by the method according to [8], and transplanting the composite sheet for retinal transplantation into the retina of a subject in need thereof.

### Advantageous Effects of Invention

With conventional retinal organoids, the excision of grafts with a major axis of about 1 mm required highly discriminating eye and skilled technique, and inspection of each organoid was necessary. Moreover, the excised grafts were non-uniform due to being dependent on the shape and size of the organoid. According to the method of the present invention, a sheet suitable for transplantation can be efficiently excised from a single large planar retinal organoid using an objective method of evaluation.

### Brief Description of Drawings

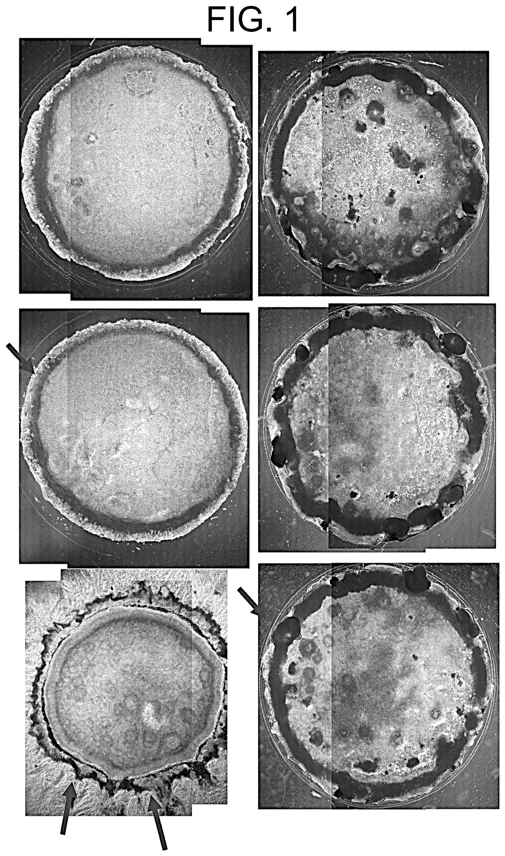

[Figure 1] Figure 1 shows bright-field microscope images. The uniformity of the sheet, the formation of organoid structures and retinal pigment cells, which are chromocytes, in the surrounding area (arrows), and retinal differentiation can be assessed.

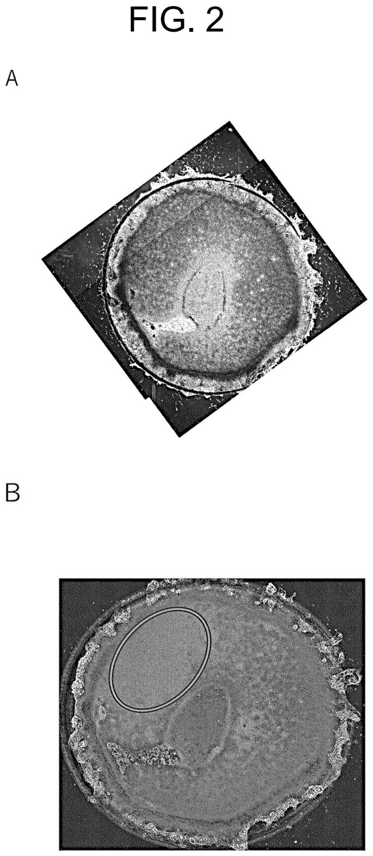

[Figure 2] Figure 2 shows phase-contrast microscope images. These allow estimation of the uniformity of the internal structure. In the uniform region (A, area enclosed by the line), a uniform retinal tissue structure can be observed in the immunostained image (B).

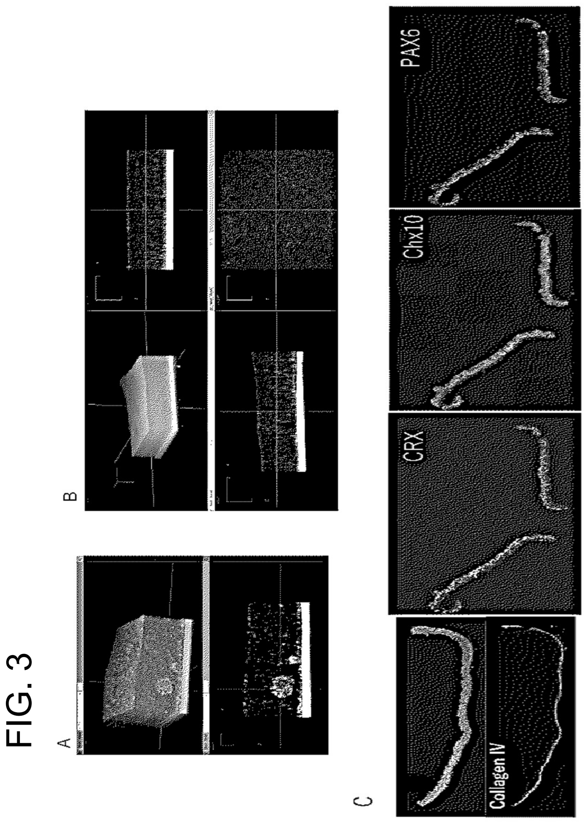

[Figure 3] Figure 3 shows OCT images. An area within the retina containing foreign structures (A) and an area free of foreign structures (B) can be distinguished. In the area free of foreign structures and with a uniform thickness, at day 60 of differentiation (C), the basal surface is positive for Collagen IV, retinal progenitor cells (Chx10) are distributed over a wide area on the parietal side, with photoreceptor progenitor cells (CRX) on the parietal side within that, and more differentiated cells (PAX6) of the inner layer of the retina on the basal membrane side.

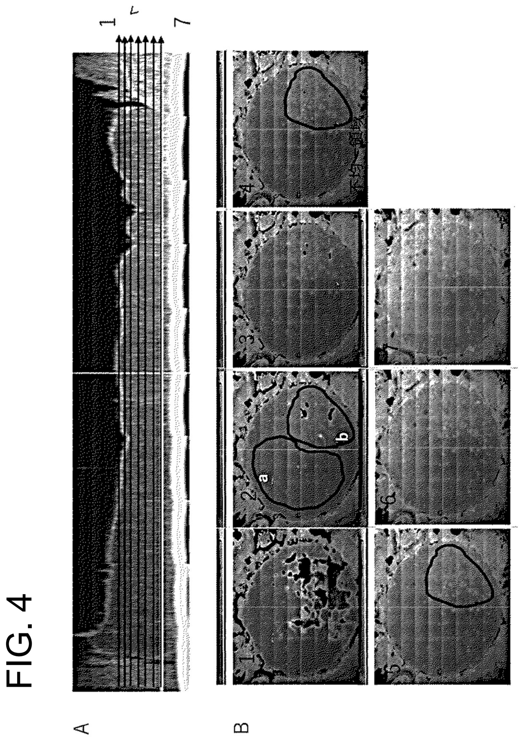

[Figure 4] Figure 4 shows the reconstruction of horizontal X-Y planar images from OCT cross-sectional images (three-dimensional images). By using X-Y planar images, the presence or absence of foreign structures within the sheet can be efficiently determined at intervals of 10 µm or less using a smaller number of sequential images. The upper left region (a) exhibits nearly uniform brightness across multiple cross-sectional images and is free of foreign structures, whereas the lower right region (b) shows uneven brightness distribution. When approaching the top surface (No. 1 side), voids can be observed in multiple spots or in a mottled pattern in the lower right region across multiple cross-sectional images, indicating that the surface is not smooth.

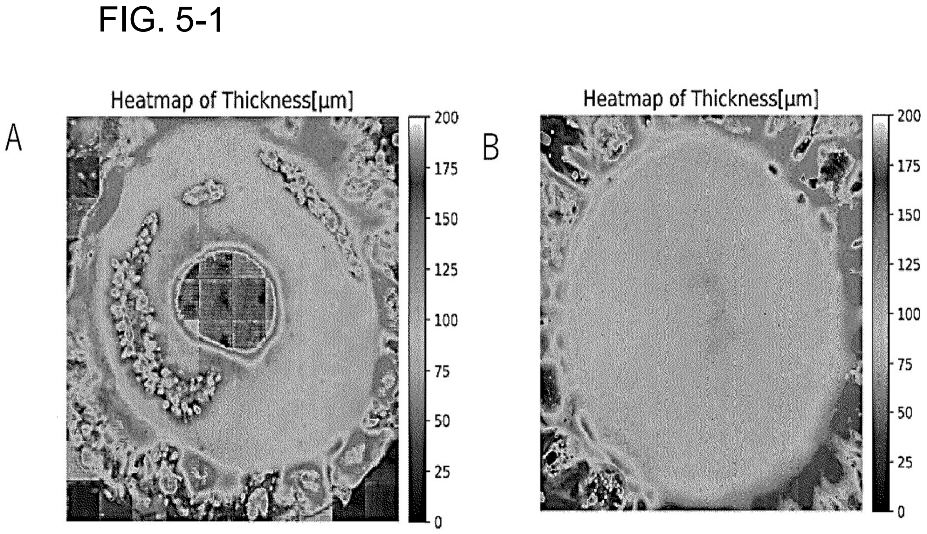

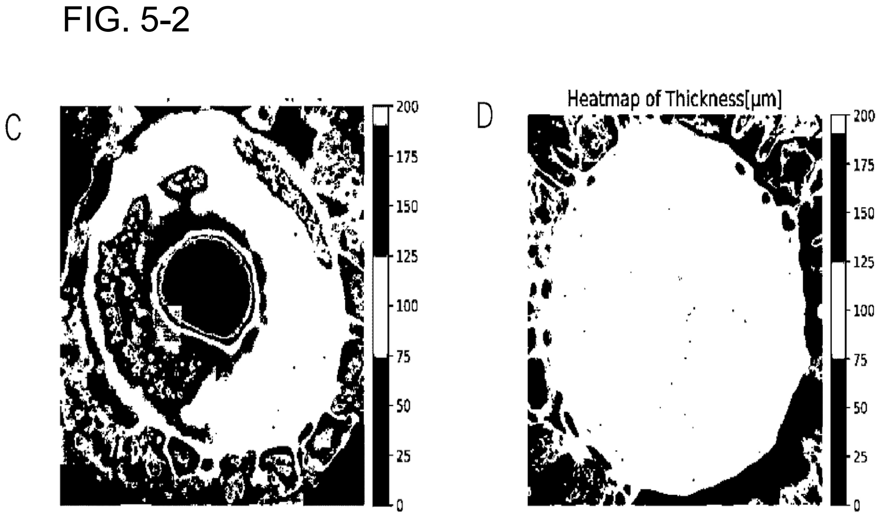

[Figure 5] Figure 5 shows heat maps (A, B) generated from OCT cross-sectional images, and figures (C, D) obtained by further binarization of the heat maps. Here, the binarization is performed such that regions with a thickness of 70 µm or more are shown in white, and regions with a thickness of less than 70 µm are shown in black (C, D). The heat maps allow determination of the thickness and the structures with uneven thickness in the organoids (A, C). In the organoids (A, C), the region suitable for transplantation is limited to the right side, whereas in the organoids (B, D), the entire area is determined to have a thickness suitable for transplantation.

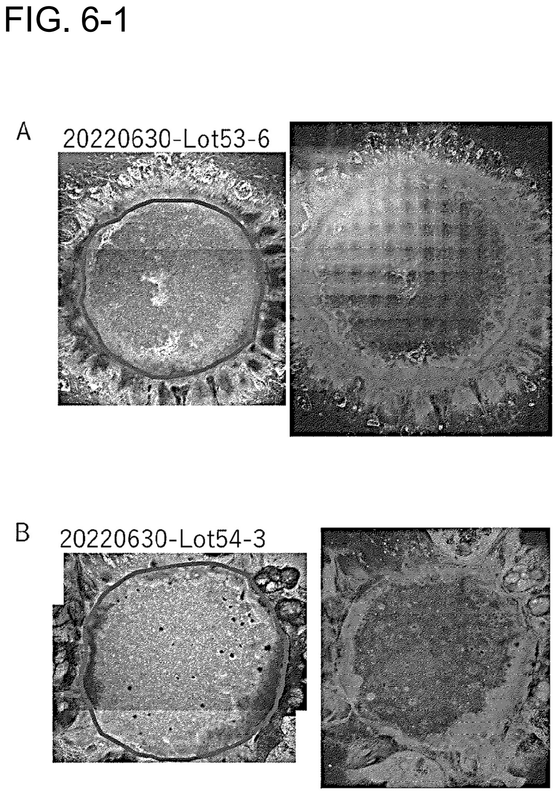

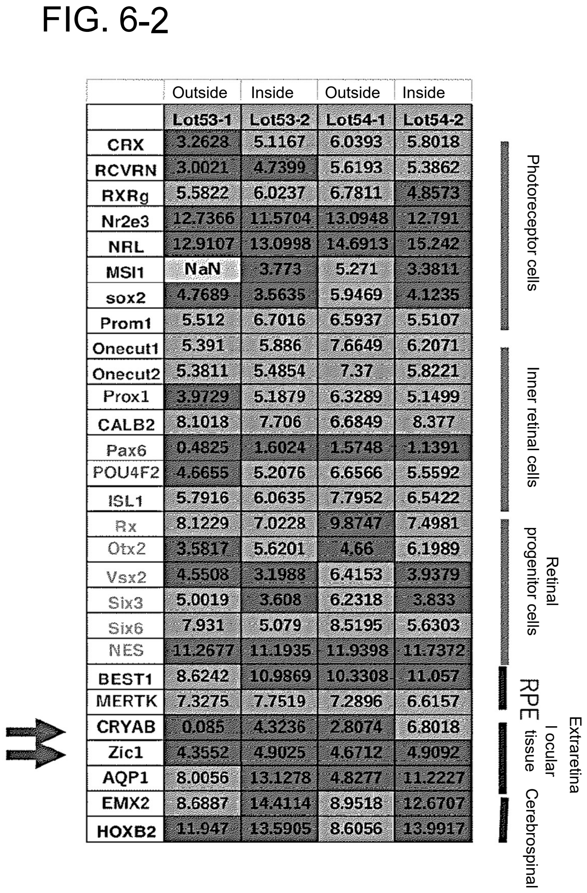

[Figure 6] Figure 6 shows the results of quantitative PCR of a thin retinal organoid with a thickness of less than 50 µm. By using a PCR panel to identify the cell groups constituting the retinal organoid, such as photoreceptor cells, retinal progenitor cells, and inner retinal cells, it is possible to assess retinal differentiation and contamination by extraretinal tissue, and determine the general composition of inner cells/photoreceptor cells within the retinal cells at 60 days of differentiation. In such thin sheets, in addition to photoreceptor cell markers, an increase in markers for non-retinal ocular tissues such as the lens and ciliary body is observed.

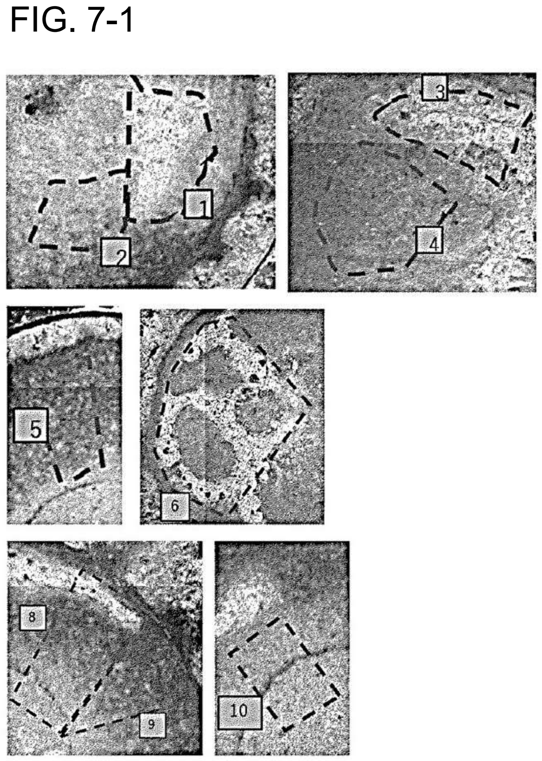

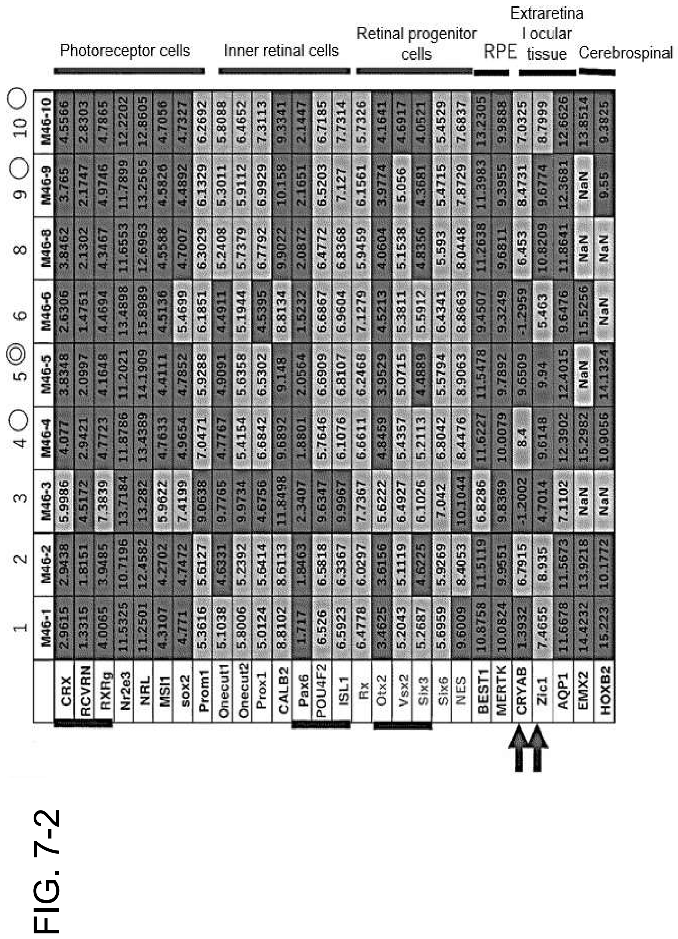

[Figure 7] Figure 7 shows the results of quantitative PCR of planar retinal organoids with 1) a uniform brightness distribution and 2) a thickness of 70 µm or more (4, 5, 9, and 10), as well as of the regions that do not satisfy either 1) or 2) (1-3 and 6-8). In the thick and uniform regions (4, 5, 9, and 10), the expression levels of marker genes for extraretinal tissues are low, and markers for each group of photoreceptor progenitor cells, photoreceptor progenitor cells, and inner retinal cells, which are components of the retinal organoids, can be confirmed in sets, indicating that the area is suitable for transplantation. The arrows are exclusion markers showing unsuitable reference values.

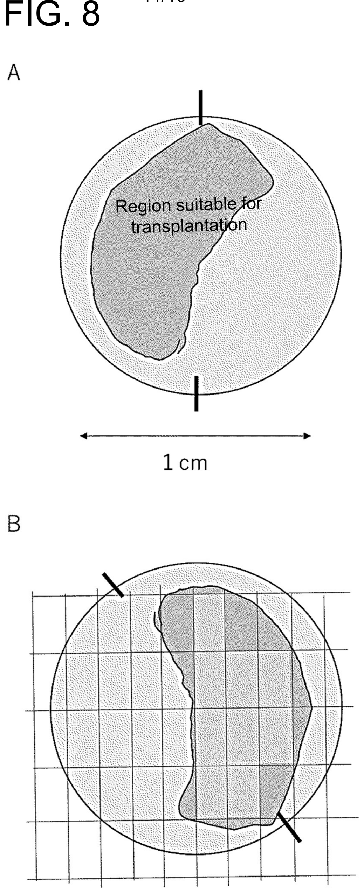

[Figure 8] Figure 8 shows an example of the excision of retinal sheets for transplantation from the region suitable for transplantation of a retinal organoid. The retinal sheets for transplantation are excised by aligning a grid to maximize the effective number of sheets within the region suitable for transplantation.

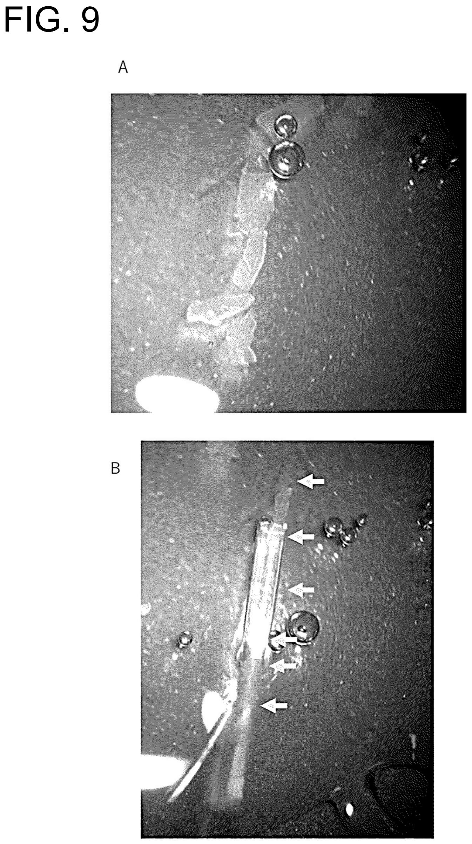

[Figure 9] Figure 9 shows a 24G cannula for transplantation filled with a plurality of retinal sheets for transplantation of about 1 x 2 mm.

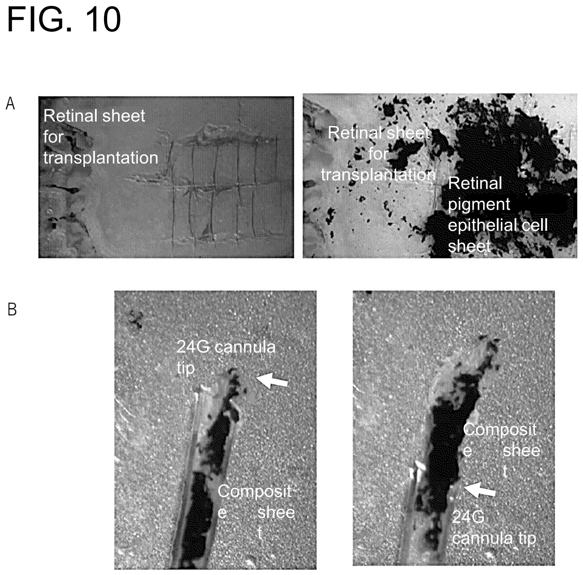

[Figure 10] Figure 10 shows a composite sheet in which a retinal sheet for transplantation is laminated onto a retinal pigment epithelial sheet using 7-fold diluted hyaluronic acid for intraocular surgery (Viscoat^{™}). The composite sheet (B), produced by laminating the retinal pigment epithelial cell sheet onto the retinal sheet for transplantation (A), can be inserted into the 24G cannula and extruded from the cannula while maintaining its composite state (C, D).

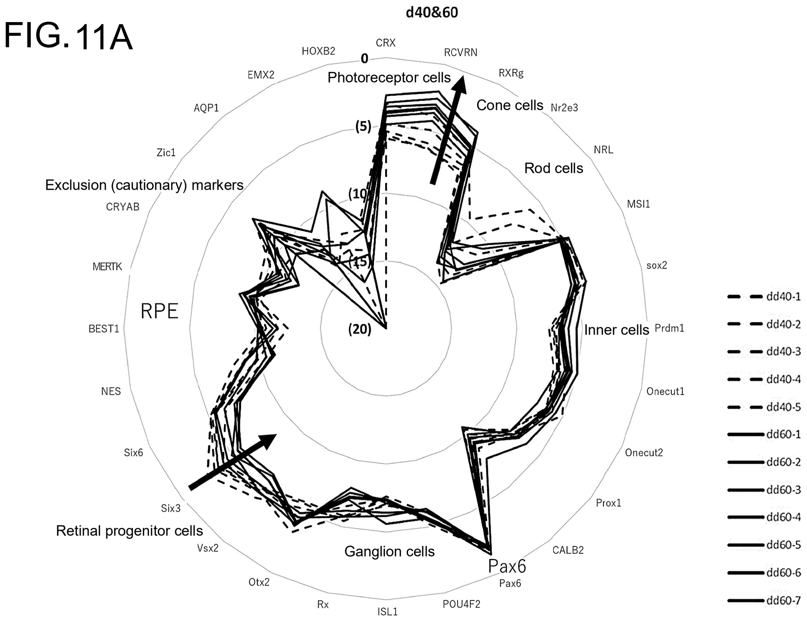

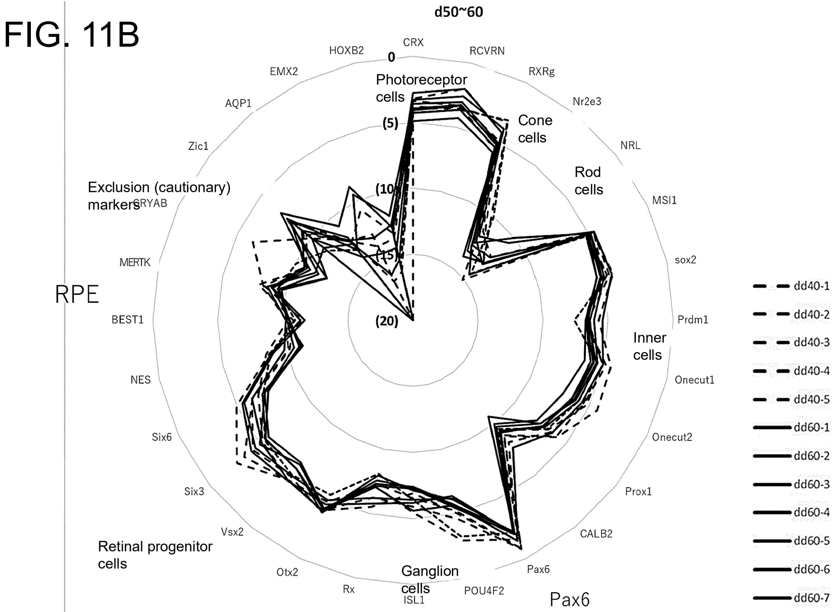

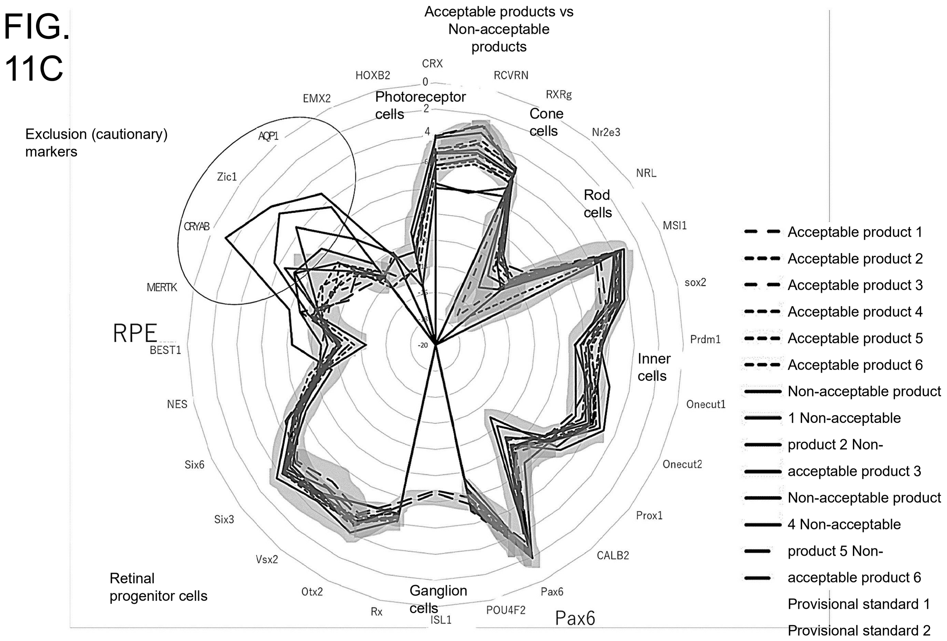

[Figure 11] Figure 11 is a radar chart showing the retinal gene expression patterns of tissue fragments excised from the same retinal organoid. A: Comparison of expression patterns at days 40 and 60 of differentiation. A decrease in the expression of retinal progenitor cell markers and an increase in the expression of photoreceptor cell markers from day 40 (dotted line) to day 60 (solid line) of differentiation can be observed (arrows). B: Comparison of expression patterns at days 50 (dotted line) and 60 (solid line) of differentiation. The expression patterns are nearly the same, allowing evaluation based on the same criteria. C: By collecting standard data, it is possible to identify a range of retinal gene expression patterns (gray zone in C) that indicate a good sheet. In this chart, the exclusion (cautionary) markers indicate extraretinal tissue cell markers (EMX2, CRYAB, Zic1, AQP1), and sheets that include regions deviating from the pattern (circled area) can be determined to have a non-acceptable profile.

### Description of Embodiments

### 1. Method for producing retinal sheet for transplantation

The present invention relates to a method for producing a retinal sheet for transplantation from a retinal organoid derived from pluripotent stem cells, the method including a step of performing optical coherence tomography on the retinal organoid derived from pluripotent stem cells to identify, as a region suitable for transplantation, a region with a thickness of 70 µm or more and free of foreign structures characterized by differences in brightness, and a step of excising a fragment from the region suitable for transplantation to obtain the retinal sheet for transplantation.

A "pluripotent stem cell" according to the present invention refers to an embryonic stem cell (hereinafter referred to as "ES cell") and a cell that potentially possesses pluripotency similar to that of ES cells. Examples of cells possessing pluripotency similar to that of ES cells include induced pluripotent stem cells (hereinafter referred to as "iPS cells"), which are somatic cells that have been reprogrammed to acquire pluripotency and self-renewal capacity.

ES cells are pluripotent stem cells that are derived from early embryos, and various established ES cell lines can be utilized. Examples of ES cell lines that can be used include, but are not limited to, clinical-grade human ES cell lines provided by the Center for Human ES Cell Research of the Institute for Life and Medical Sciences, Kyoto University, and human ES cell lines (KhES-1, KhES-1_Crx: :Venus, and KhES-1_Rx: :Venus) provided by RIKEN BRC.

As the iPS cells, not only iPS cells induced from the patient's own somatic cells but also various iPS cell lines can be utilized. Examples of iPS cell lines that can be used include, but are not limited to, various iPS stock cell lines provided by the CiRA Foundation, Kyoto University, and iPS cell lines derived from healthy donors provided by RIKEN BRC, such as KVs09 and CLs23.

The species of pluripotent stem cells is not limited and is appropriately selected according to the recipient. In the case of a retinal sheet for human transplantation, cells derived from primates, particularly those derived from monkeys or humans, are preferred, and human-derived pluripotent stem cells are more preferred.

A "retinal organoid" refers to a three-dimensional retinal tissue produced ex vivo. Accordingly, "retinal organoid derived from pluripotent stem cells" refers to a three-dimensional retinal tissue obtained by inducing the differentiation of pluripotent stem cells ex vivo.

The method for producing a retinal organoid from pluripotent stem cells is not limited, and any method may be used, including suspension culture methods such as the SFEBq method, and the known methods described in WO2015/025967A1, WO2019/217630A1, WO2013/077425A1, WO2022/239868A1, WO2020/249935A1, JP2013-128475A, WO2022/138803A1, JP2021-118733A, WO2017/183732A1, WO2020/249935, and WO2022/054925A1.

The retinal organoid preferably has a diameter of about 1 cm or more so that a plurality of grafts can be excised therefrom. Herein, a diameter of 1 cm or more refers to the length of the portion corresponding to the diameter in the case where the retinal organoid has a shape close to a circle, or to the major axis in the case where the retinal organoid has a shape close to an ellipse.

The retinal organoid is preferably a planar retinal organoid. The planar retinal organoid can be produced by culturing pluripotent stem cells on a suitable substrate in a planar manner. According to this method, it is possible to control the morphology and size of the tissue, which is difficult with a suspension culture method. The method for producing a planar retinal organoid will be described in detail in "2. Preparation of Retinal Organoid."

In the present invention, optical coherence tomography (OCT) is performed on the retinal organoid described above to identify regions suitable for transplantation. "Optical coherence tomography" is a technique that utilizes the coherence of light to create an image of the internal microstructure of the measured object, and allows for high-speed, high-resolution, non-invasive, real-time observation of the structure of the retinal organoid. Herein, OCT encompasses Spectral domain OCT, Swept-source OCT, full-field OCT, adaptive optics OCT, and the like. The region suitable for transplantation must be a region that can be confirmed by OCT to have a uniform thickness and to be free of foreign structures. For the same purpose, a confocal imaging system using infrared to near-infrared light, an acoustic microscope, or the like can also be used as a means for observing the internal structure of the retinal organoid by varying the depth.

As used herein, the "region suitable for transplantation" refers to a region within the retinal organoid that is suitable for transplantation into the retina. The region suitable for transplantation is a region that has been confirmed by OCT to have a thickness of at least 70 µm or more, preferably 70 to 175 µm, or 80 µm or more, more preferably 80 to 150 µm. The thickness of the retinal organoid can be confirmed by an OCT cross-sectional image (three-dimensional image), a horizontal planar image reconstructed from an OCT cross-sectional image, or the like. In particular, the thickness of the retinal organoid can be readily confirmed by using a heat map produced from OCT cross-sectional images, and the suitable range can be easily narrowed down by further using a binarized image.

The region suitable for transplantation must be a region that, in addition to having a thickness of 70 µm or more, has been confirmed by OCT to be free of foreign structures. A "foreign structure" is characterized by a high brightness differing from the average brightness distribution of other uniform regions in the OCT image brightness distribution. Since the retinal organoid has a layered structure, it is preferable to determine the presence or absence of foreign structures by sequentially extracting a plurality of horizontal X-Y planar images reconstructed from OCT images along the Z direction, for example, at intervals of about 10 µm or less, and evaluating them (see Figure 4).

In the present specification, "about" indicates a value that varies up to ±10%, ±8%, ±6%, ±5%, ±4%, ±3%, ±2%, or ±1% relative to the reference value. Preferably, the term "about" indicates a range of ±10%, ±5%, or ±1% relative to the reference value.

The region suitable for transplantation can be efficiently identified by excluding the regions that are not suitable for transplantation (regions unsuitable for transplantation) or the retinal organoids that are not suitable for transplantation by using bright-field microscopy and/or phase-contrast microscopy in addition to the various types of OCT.

"Bright-field microscopy" is suitable for assessing the overall condition of the retinal organoid. When differentiation into the retina is observed, the organoid exhibits the formation of characteristic retinal pigment cells (RPE) or spherical three-dimensional organoid-like structures at the periphery, and therefore the presence of these structures allows confirmation of whether the organoid has appropriately differentiated into retina. In addition, since the thicker areas are observed in a color different from the thinner areas (for example, as light brown), it is also possible to roughly determine the overall uniformity and thickness. Bright-field microscopy allows exclusion of the regions where retinal differentiation is not observed, or the regions that are non-uniform and thin, from examination by OCT. Furthermore, the differentiation status can be recorded over time by bright-field microscopy, and the progress thereof can also be followed to confirm that differentiation is occurring in a favorable manner.

"Phase-contrast microscopy" is an optical microscopy technique that allows observation by converting phase differences of light into contrast. Since it allows observation of a subject without staining and in a non-invasive manner, it is useful for the observation of cells and tissues. Phase-contrast microscopy also allows detailed observation of internal structures that cannot be observed by bright-field microscopy, and therefore allows confirmation of the presence or absence of foreign structures within the retinal organoid, as well as the rough uniformity and thickness thereof. Phase-contrast microscopy allows exclusion of the regions in which foreign structures are observed, or the regions that are non-uniform and thin, from examination by OCT.

The excision of a fragment for transplantation from the region suitable for transplantation can be performed by a known method. For example, a grid corresponding to the size of the fragment for transplantation is set on the retinal organoid, and aligned so that the number of fragments for transplantation obtainable from the region suitable for transplantation (effective number) is maximized, then the fragments are excised along the grid.

The excised fragment preferably has a size that can be loaded into a cannula for transplantation, with a length of 1.0 to 10.0 mm in the long axis direction and a length of 0.5 to 2.0 mm in the short axis direction. For example, when using a 24G cannula, the length in the minor axis direction is about 0.5 to about 1.0 mm, while the length in the major axis direction can be freely set. When using a 18G cannula, the length in the minor axis direction can also be extended up to about 2.0 mm. By filling the cannula with a low-viscosity viscoelastic substance, such as a 7-fold diluted hyaluronic acid for ophthalmic surgery (e.g., Viscoat), it is possible to load a plurality of fragments for transplantation. Particularly, when using a 24G cannula, it is possible to transplant the fragments to the intended area with a small volume of medium, within about 10 µL, without making a large incision in the retina.

The retinal sheet for transplantation obtained by the method of the present invention is composed of a retinal organoid in which the ratio of retinal cells (including retinal progenitor cells) to all cells is about 90% or more, and more preferably about 95% or more. The retinal sheet for transplantation consisting of such retinal organoid can be easily obtained without relying on the skill of an expert, by a method using OCT, or OCT in combination with bright-field microscopy and/or phase-contrast microscopy, as described above.

The method of the present invention may further include a step of performing quantitative PCR on the excised fragment (retinal sheet for transplantation) to examine its gene expression pattern and confirm that it is a retinal graft of the desired quality. The confirmation of the quality of the retinal sheet for transplantation by such quantitative PCR will be described in detail in "3. Quality Control of Retinal Sheet for Transplantation."

### 2. Preparation of Planar Retinal Organoid

Examples of the method for producing a retinal organoid from pluripotent stem cells include a method of inducing differentiation of pluripotent stem cells into a retinal tissue in a region A on a culture substrate having, on its surface, a region A having cell adhesiveness and a region B adjacent to at least a part of the region A and having cell adhesiveness lower than the cell adhesiveness of the region A. Hereinafter, culture on a substrate having such a specific pattern is referred to as "patterned culture."

The region A is a region that can maintain pluripotent stem cells in adhesive culture. The region B is a region having cell adhesiveness lower than that of the region A so that, when pluripotent stem cells are seeded on the region A, the cells are prevented from extending from the region A to the region B adjacent to the region A during the induction of retinal tissue differentiation (i.e. the period until differentiation to a retinal tissue is confirmed). The cell adhesiveness can be compared by seeding pluripotent stem cells of an excess amount relative to an area (e.g. 5×10⁵ to 10×10⁵ cells/cm²) and comparing the percentage of the area to which the cells adhere after 24 hours culture at 37°C and 5% CO₂. The cell adhesiveness is typically evaluated after the medium is removed after the 24 hours culture and the cells are washed with a medium or buffer to remove unadhered cells.

The region A may be, for example, a region where, when pluripotent stem cells of an excess amount relative to its area (e.g. 5×10⁵ to 10×10⁵ cells/cm²) are seeded and cultured at 37°C and 5% CO₂ for 24 hours, the cells adhere to 70% or more, 80% or more, or 90% or more of the area after the 24 hours.

In some embodiments, the region B is a region having no cell adhesiveness. The region having no cell adhesiveness refers to a region that does not have sufficient cell adhesiveness to maintain pluripotent stem cells in adhesive culture. The region having no cell adhesiveness may be, for example, a region where, when pluripotent stem cells of an excess amount relative to its area (e.g. 5×10⁵ to 10×10⁵ cells/cm²) are seeded and cultured at 37°C and 5% CO₂ for 24 hours, the cells adhere to 30% or less, 20% or less, or 10% or less of the area after the 24 hours.

The culture substrate may be made of any material as long as it can have the region A and the region B on its surface. For example, the culture substrate may be made of an inorganic material such as metal, glass, or silicone, or an organic material such as plastic. In some embodiments, the culture substrate is made of glass or plastic, especially polystyrene resin.

The culture substrate is not particularly limited, and may have any shape commonly used for cell culture. The culture substrate may be, for example, a culture vessel such as a petri dish, plate, bottle, chamber, or multi-well plate, or may be a film or porous membrane.

The region A may be a region of the exposed surface of a cell-adhesive culture substrate, or a region of the surface of a culture substrate coated with a cell-adhesive material to make the surface cell-adhesive or to enhance the cell adhesiveness of the surface. Examples of cell-adhesive materials include positively charged polymers such as poly L-lysine and poly L-ornithine; laminin; collagens such as type I collagen, type II collagen, type III collagen, type IV collagen, type V collagen, and type VII collagen; tenascin; fibrillin; fibronectin; vitronectin; elastin; entactin; proteoglycans composed of sulfated glycosaminoglycans such as chondroitin sulfate, heparan sulfate, keratan sulfate, and dermatan sulfate and core proteins; glycosaminoglycans such as chondroitin sulfate, heparan sulfate, keratan sulfate, dermatan sulfate, and hyaluronic acid; Synthemax^{®} (a vitronectin derivative) and Matrigel^{®}.

In some embodiments, the region A is coated with laminin. The laminin is not limited, and laminin 111, laminin 121, laminin 211, laminin 213, laminin 222, laminin 311 (laminin 3A11), laminin 332 (laminin 3A32), laminin 321 (laminin 3A21), laminin 3B32, laminin 411, laminin 421, laminin 423, laminin 521, laminin 522, laminin 523, or fragments thereof (for example, laminin 211-E8, laminin 311-E8, laminin 411-E8, and laminin 511-E8, which are E8 fragments) can be used. In some embodiments, laminin is laminin 511 or a fragment thereof (for example, laminin 511-E8 fragment).

The region B may be a region of the exposed surface of a culture substrate having cell adhesiveness lower than that of the region A, or a region of the surface of a culture substrate coated with a material to make the region less cell-adhesive than the region A. In some embodiments, the region B is coated with a non-cell-adhesive material. Examples of non-cell-adhesive materials include MPC (2-methacryloyloxyethyl phosphorylcholine) polymers; celluloses such as methyl cellulose, hydroxyethyl cellulose, hydroxypropyl cellulose, hydroxypropyl methyl cellulose, and sodium carboxymethyl cellulose; polyethylene oxide; carboxyvinyl polymers; polyvinylpyrrolidone; polyethylene glycol; polyamides such as polyacrylamide and poly-N-isopropylacrylamide; polysaccharides such as chitin, chitosan, hyaluronic acid, alginic acid, starch, pectin, carrageenan, guar gum, gum arabic, and dextran; albumin and their derivatives. In some embodiments, the non-cell-adhesive material is an MPC polymer.

The shape of the region A is not limited. In some embodiments, the region A has a circular shape. As used herein, a circular shape refers to a shape that is recognized as substantially circular in the art, including a perfect circle. The area of the region A may be, but is not limited to, 0.5 to 10 cm², 0.7 to 10 cm², 1 to 10 cm², 0.5 to 5 cm², 0.7 to 5 cm², or 1 to 5 cm².

The regions A and B may be formed on the surface of a culture substrate by any method used for cell patterning. The regions A and B for example can be formed on the surface of a culture substrate by techniques such as soft lithography, photolithography, and 3D printing.

For example, the regions A and B may be formed on a culture substrate by preparing a sheet with a hole of the shape and size of the region A using a 3D printed mold, covering the surface of the culture substrate with the sheet, coating the portion of the surface not covered with the sheet with a cell-adhesive material, and then removing the sheet. Alternatively, the regions A and B may be formed on a culture substrate by preparing a sheet of the shape and size of the region A, placing the sheet on the surface of the culture substrate, coating the portion of the surface not covered with the sheet with a non-cell-adhesive material, removing the sheet, and coating the portion of the surface covered with the sheet with a cell-adhesive material. The sheet may be prepared from a biocompatible material such as polydimethylsiloxane (PDMS), polyethylene glycol hydrogel, or agarose gel. The coating can be performed, for example, by bringing the culture substrate into contact with a solution of a cell-adhesive or non-cell-adhesive material and allowing it to react with the solution at 37°C or room temperature for the required time (e.g., 1 hour or more). The concentration of the cell-adhesive or non-cell-adhesive material can be appropriately determined by those skilled in the art, and, for example, for laminin, may be 0.1 to 1 µg/cm², 0.1 to 0.5 µg/cm², or about 0.25 µg/cm².

In the present specification, the culture substrate may have a convex portion (also called a pillar) on the surface and the convex portion may have the region A on its upper surface. The shape of the convex portion may be, but is not limited to, a circular cylinder or a prism.

The pluripotent stem cells are seeded on a culture substrate at a cell number so that the cells occupy 70% or more of the region A. The cells may be seeded only in the region A or on the entire region of the culture substrate including the region A and the region B. For example, when the region B is a non-cell-adhesive region, the cells can be seeded on the entire region of the culture substrate. For example, the pluripotent stem cells may be seeded at 5×10⁵ to 10×10⁵ cells/cm². Typically, pluripotent stem cells are collected and dispersed with a cell dispersing solution containing an enzyme (e.g., trypsin, collagenase, hyaluronidase, elastase, pronase, DNase, or papain) and/or a chelating agent (e.g., ethylenediaminetetraacetic acid (EDTA)), and suspended in a desired medium. Then, the resulting cell suspension is added onto the culture substrate. As the cell dispersing solution, for example, a commercial product such as TrypLE^{™} Select (Thermo Fisher Scientific) or TrypLE^{™} Express (Thermo Fisher Scientific) may be used.

Differentiation induction of pluripotent stem cells into a retinal tissue is performed by adhesive culture on the culture substrate described above. The differentiation induction of pluripotent stem cells into a retinal tissue may be started after pluripotent stem cells seeded on a culture substrate are cultured for a certain period of time in a medium containing a factor for maintaining undifferentiated state. In the present disclosure, the time when the culturing in a medium that does not contain a factor for maintaining undifferentiated state is started is defined as the time when the differentiation induction into a retinal tissue is started.

The differentiation induction of pluripotent stem cells into a retinal tissue can be performed according to a known method as described above. In some embodiments, the differentiation induction of pluripotent stem cells into a retinal tissue comprises culturing pluripotent stem cells in a medium containing a BMP signaling activator. In further embodiments, the differentiation induction of pluripotent stem cells into a retinal tissue comprises the following steps:
(1) culturing pluripotent stem cells in a medium containing a BMP signaling inhibitor; and
(2) culturing the cells obtained by the step (1) in a medium containing a BMP signaling activator.

The medium used can be prepared by using a medium commonly used for culturing animal cells as a basal medium. Examples of basal media include IMDM, DMEM, F-12, DMEM/F12, IMDM/F12, BME, BGJb, CMRL 1066, Glasgow MEM (GMEM), Improved MEM Zinc Option, Medium 199, Eagle MEM, alpha MEM, Ham's medium, RPMI 1640, Fischer's medium, and mixtures of thereof.

To the above basal medium, one or more components selected from serum, serum substitutes, growth factors, factors for maintaining undifferentiated state, cytokines, insulin, fatty acids, lipids, vitamins, amino acids (e.g., nonessential amino acids, retinoids, glutamine, taurine), antioxidants, 2-mercaptoethanol, 1-thioglycerol, antibiotics, buffers, and inorganic salts can be added as needed.

The medium may be a commercially available medium that contains any of the above components in a basal medium, such as Ham's F-12 Nutrient Mix, GlutaMAX^{™} Supplement (Thermo Fisher Scientific) or DMEM/F-12, GlutaMAX^{™} Supplement (Thermo Fisher Scientific).

The medium is preferably a serum-free medium. The differentiation induction is preferably performed under feeder-free conditions, and more preferably under xeno-free conditions.

The "BMP signaling inhibitor" is not limited as long as it is a substance that inhibits signaling by BMP. Examples of BMP signaling inhibitors include LDN-193189 (4-[6-(4-Piperazin-1-ylphenyl)pyrazolo[1,5-a]pyrimidin-3-yl]quinoline), Dorsomorphin (6-[4-[2-(1-Piperidinyl)ethoxy]phenyl]-3-(4-pyridinyl)-pyrazolo[1,5-a]pyrimidine), and DMH1 (4-(6-(4-isopropoxyphenyl)pyrazolo[1,5-a]pyrimidin-3-yl)quinoline). In some embodiments, the BMP signaling inhibitor is LDN-193189. The concentration of a BMP signaling inhibitor is determined according to the inhibitor to be used, and may be 1 to 1000 nM, 10 to 500 nM, 30 to 300 nM, or about 100 nM, or a concentration providing a BMP inhibitory activity equivalent to that of LDN-193189 at the concentration as mentioned above.

The period of culturing in a medium containing a BMP signaling inhibitor may be, but is not limited to, 1 to 10 days, 2 to 9 days, 3 to 7 days, 4 to 6 days, or about 5 days.

The "BMP signaling activator" is not limited as long as it is a substance that activates signaling by BMP. Examples of BMP signaling activators include BMP2, BMP4, BMP7, BMP12 (GDF7) and fragments thereof, and anti-BMP receptor antibodies. In some embodiments, the BMP signaling activator is BMP4. The concentration of a BMP signaling activator is determined according to the activator to be used, and may be 0.01 to 1000 nM, 0.1 to 100 nM, 1 to 10 nM, 1 to 3 nM, or about 1.5 nM, or a concentration providing a BMP activity equivalent to that of BMP4 at the concentration as mentioned above.

The cells are cultured in a medium containing a BMP signaling activator for a period necessary for differentiation into a retinal tissue. The period of culturing may be, but is not limited to, 1 to 30 days, 2 to 20 days, 3 to 15 days, 4 to 12 days, or 5 to 10 days (e.g., 5, 6, 7, 8, 9, or 10 days). The concentration of a BMP signaling activator may be constant or varied during the culturing period. For example, the concentration of a BMP signaling activator may be decreased stepwise at a rate of 40 to 60% reduction per 2 to 4 days. For example, the concentration of a BMP signaling activator in a medium may be decreased stepwise by replacing a portion (e.g., half) of the medium containing the BMP signaling activator with a medium not containing the BMP signaling activator every 2, 3, or 4 days after the start of culturing in the medium containing the BMP signaling activator.

In some embodiments, the medium containing a BMP signaling inhibitor or BMP signaling activator is prepared from a 1:1 mixture of F-12 medium and IMDM medium supplemented with KnockOut^{™} Serum Replacement (Thermo Fisher Scientific) (e.g., 0.5% to 30%, 1% to 20%, or 10%), Chemically Defined Lipid Concentrate (Thermo Fisher Scientific), BSA, and 1-thioglycerol.

After the culturing in a medium containing a BMP signaling activator, the medium may be replaced with a medium containing no BMP signaling activator to continue the culturing. This period of culturing may be, but is not limited to, 1 to 100 days, 10 to 90 days, 20 to 80 days, 30 to 70 days, 40 to 60 days, or about 50 days.

The cells may be cultured in a medium containing a Wnt signaling inhibitor during a part or the whole of the period of culturing in a medium containing a BMP signaling activator. Examples of Wnt signaling inhibitors include CKI-7 (N-(2-Aminoethyl)-5-chloro-8-isoquinolinesulfonamide), D4476 (4-(4-(2,3-Dihydrobenzo[1,4]dioxin-6-yl)-5-pyridin-2-yl-1H-imidazol-2-yl)benzamide), IWR-1-endo (IWR1e) (4-[(3aR,4S,7R,7aS)-1,3,3a,4,7,7a-Hexahydro-1,3-dioxo-4,7-methano-2H-isoindol-2-yl]-N-8-quinolinylbenzamide), and IWP-2 (N-(6-methyl-2-benzothiazolyl)-2-[(3,4,6,7-tetrahydro-4-oxo-3-phenylthieno[3,2-d]pyrimidin-2-yl)thio]-acetamide). The concentration of a Wnt signaling inhibitor is determined according to the inhibitor to be used, and may be 0.1 to 100 µM, 0.3 to 30 µM, or 1 µM to 10 µM.

For the preparation of the retinal sheet for transplantation as mentioned above, it is preferable to use a planar retinal organoid that is about 40 to 90 days, more preferably about 45 to 80 days, and particularly preferably about 50 to 70 days, from the start of differentiation of pluripotent stem cells. This is because sufficient retinal differentiation from pluripotent stem cells can be expected for a retinal organoid at this stage. A planar retinal organoid at about 60 days of differentiation is characterized by a bilayer structure containing primarily more immature retinal progenitor cells (Chx10- or RX-positive) on the parietal side, and more differentiated inner retinal cells such as ganglion cells (BRN3B, Pax6, ISL1) on the basal membrane side.

### 3. Quality Confirmation of Retinal Cell Sheet

The fact that the retinal sheet for transplantation prepared by the method of the present invention has the desired quality can be confirmed by performing quantitative PCR in accordance with previous reports (e.g., WO2020/184720), and displaying the gene expression pattern, for example, on a radar chart. Alternatively, it can be confirmed that the suitable region is a retinal organoid containing photoreceptor progenitor cells by performing immunostaining, single-cell analysis, analyses using flow cytometry, or analyses based on machine learning or deep learning using these analyses as training data.

Quantitative PCR allows confirmation that the expression levels of markers for extraretinal cells are low and that the expression levels of markers for retinal cells are high. This confirmation includes not only evaluation based on the expression levels of specific markers, but also evaluation based on the expression patterns or profiles of a plurality of markers.

Specifically, it is necessary to confirm, based on the gene expression pattern described above, that the retinal sheet for transplantation contains markers of a plurality of constituent cell groups as indicators that it is a retinal organoid, and that it does not contain extraretinal tissue cells. Here, retinal cells and their progenitor cells at days 40 to 80 of differentiation include retinal progenitor cells, photoreceptor progenitor cells (including cone and rod progenitor cells), inner retinal cells (horizontal cells, amacrine cells, and retinal ganglion cells), and retinal pigment epithelial cells; whereas extraretinal tissue cells are cells other than retinal cells, and specifically include lens cells, ciliary body cells, and brain neurons. For example, in a single-cell analysis of transplant tissue around 60 days of differentiation, it is possible to identify populations of retinal progenitor cells, as well as some early-differentiating inner retinal cells and photoreceptor progenitor cells.

Examples of retinal progenitor cell markers include RX, Vsx2, Otx2, Six3, and Six6. Examples of photoreceptor progenitor cell markers include Recoverin, Rxr-gamma, CRX, Nr2e3, Nrl, MSI1, Sox2, and Prom1. Of these, examples of markers that particularly predict later ratios of cone cells include Rxr-gamma, ARR3, and THRB. Examples of markers for inner retinal cells include Calbindin, Onecut1, Onecut2, Prox1, Chx10, POU4F2, and ISL1. Examples of markers specific to retinal pigment epithelial (RPE) cells include MITF, TTR, BEST1, RPE65, and MERTK.

Examples of markers for extraretinal tissue cells include CRYAB, EMX2, and s100A10, and examples of markers for ciliary body, for which contamination below a certain level is acceptable, include Zic1, AQP1, and MSX1.

The retinal sheet for transplantation of the present invention can be evaluated and determined to have the desired quality if, of the retinal cell markers described above, the expression levels of photoreceptor progenitor cell markers are high, the expression of inner retinal cell markers and retinal progenitor cell markers is confirmed, and the expression levels of extraretinal tissue cell markers are low or below the detection limit, in quantitative PCR. In particular, it is preferable to use, as indicators, the expression of one or more selected from the photoreceptor progenitor cell markers Crx, Recoverin, and Rxr-gamma, as well as the expression of one or more selected from the extraretinal tissue cell markers CRYAB, Zic1, and EMX2. In addition, not only the expression levels of each individual gene but also the "relative expression pattern" of these genes can serve as important markers for determining that it is retinal, and for defining its characteristics and quality. For example, by plotting each gene on a radar chart, it is possible to confirm the expression levels of each gene group and the relative expression ratios between any two genes (see 4 of Example 1 described below and Figure 11). In addition, by identifying the deviations from the pattern on the chart in addition to the numerical values of the individual genes for the exclusion markers such as extraretinal tissue cell markers, as well as the cautionary markers such as ciliary body markers, for which contamination below a certain level is acceptable, it is possible to determine whether an outlier value in a single gene is an isolated event or part of the overall expression pattern, thus potentially allowing for more reliable assessment.

The confirmation of markers can be carried out by a known method. For example, total RNA is extracted from each sample with RNeasy Micro Kits (Qiagen), and cDNA is synthesized with SuperScript^{®} III Reverse Transcriptase Kits (Invitrogen). The synthesized cDNA sample is diluted and subjected to quantitative PCR for each primer using TB Green^{®} Premix DimerEraser^{™} (Takara). For the evaluation, for example, each ΔCT value is averaged using an internal control (e.g., GAPDH and ARP) to determine the ΔCT value of the corresponding primer, and then the ΔCT values are compared with each other. The assessment is performed, for example, in accordance with predetermined criteria such that a ΔCT value of 5 or less is determined to be strong expression, and a ΔCT value of 5 to 7 is determined to be expression. Table 1 shows an example of primers used for quantitative PCR.

**[Table 1]**

| Marker | Primer | Sequence | SEQ ID # |
|---|---|---|---|
| GAPDH | 273 hGAPDH realtime fw | ACATCGCTCAGACACCATG | 1 |
| | 274 hGAPDH realtime rw | TGTAGTTGAGGTCAATGAAGGG | 2 |
| ARP | 275 hARP realtime fw | CGACCTGGAAGTCCAACTAC | 3 |
| | 276 hARP realtime rw | ATCTGCTGCATCTGCTTG | 4 |
| CRX | 19 CRX realtime fw1 | CCCCACTATTCTGTCAACGC | 5 |
| | 20 CRX realtime rv1 | AAACAGTGCCTCCAGCTC | 6 |
| NES | 27 NES realtime fw1 | TGCGGGCTACTGAAAAGTTC | 7 |
| | 28 NES realtime rv1 | GGCTGAGGGACATCTTGAG | 8 |
| NRL | 25 NRL realtime fw2 | AGGAGACAGGAGCCCAG | 9 |
| | 26 NRL realtime rv2 | GCACAGACATCGAGACCAG | 10 |
| RCVRN | 17 RCVRN realtime fw2 | TAACGGGACCATCAGCAAG | 11 |
| | 18 RCVRN realtime rv2 | TCCTCGGGAGTGATCATTTTG | 12 |
| Six3 | 11 SIX3 realtime fw1 | CAACCCCAGCAAGAAACG | 13 |
| | 12 SIX3 realtime rv1 | CTCGGTCCAATGGCCTG | 14 |
| Six6 | 13 SIX6 realtime fw1 | GACAAAGGGACCGAGCG | 15 |
| | 14 SIX6 realtime rv1 | CTTGCTGGATAGACTGGCG | 16 |
| Vsx2 | 7 VSX2 realtime fw1 | ACCAGACCAAGAAACGGAAG | 17 |
| | 8 VSX2 realtime rv1 | TTGAATGCCTTCTCCAGCTC | 18 |
| Zic1 | 33 ZIC1 realtime fw1 | CCTACACGCATCCCAGTTC | 19 |
| | 34 ZIC1 realtime rv1 | TTGTGGTCGGGTTGTCTG | 20 |
| AQP1 | 63 AQP1 realtime fw2 | CTTCCGTGCCCTCATGTAC | 21 |
| | 64 AQP1 realtime rv2 | AGTTCACACCATCAGCCAG | 22 |
| EMX2 | 51 EMX2 realtime fw2 | TGGAACACGCCTTTGAGAAG | 23 |
| | 52 EMX2 realtime rv2 | CCAGCTTCTGCCTTTTGAAC | 24 |
| HOXB2 | 59 HOXB2 realtime fw1 | AGAAACCCAGCCAATCCG | 25 |
| | 60 HOXB2 realtime rv1 | TGTTGGTGTAAGCCGTGC | 26 |
| Rx | 295 Rx qPCR set1_F | CGTTCGAGAAGTCCCACTAC | 27 |
| | 296 Rx qPCR set1_R | GACACTTCCAGCTTCTCCTG | 28 |
| OTX2 | 439 hOTX2_realtime fw2 | TCACTCGCCACATCTACTTTG | 29 |
| | 440 hOTX2_realtime rw2 | CAGGTTCAGAGTCCTTGGTG | 30 |
| RXR γ | 35_RXR γ_q3F | CACCCAGCTCTCAGCTAAAT | 31 |
| | 36_RXR γ _q3R | GGTGGATGGGTAGTTCATGTT | 32 |
| Prox1 | 27_Prox1_q4F | CCTGATCAGAGAGCAGGAAATG | 33 |
| | 28_Prox1_q4R | CCTTCCGGTTGTAAGGAGTTT | 34 |
| Pax6 | 21_Pax6_q1F | GCGGAAGCTGCAAAGAAATAG | 35 |
| | 22_Pax6_q1R | GGGCAAACACATCTGGATAATG | 36 |
| SOX2 | 49_SOX2_q1F | GGAGAGTAAGAAACAGCATGGA | 37 |
| | 50_SOX2_q1R | GTGGATGGGATTGGTGTTCT | 38 |
| Nr2e3 | 53_Nr2e3_q1F | CCAGGCCGAAGTTCCATTTA | 39 |
| | 54_Nr2e3_q1R | CCACCCAGAAGTAACCACTATTC | 40 |
| ISL1 | 87_ISL1_q4F | GTCCTCTCAACTTCCAGATACAC | 41 |
| | 88_ISL1_q4R | TTCCCACTTTCTCCAACAGG | 42 |
| ISL1 | 85_ISL1_q3F | CGGCTTAAATTGGACTCCTAGAT | 43 |
| | 86_ISL1_q3R | GCTGTGGCTAAGTGGGAAATA | 44 |
| POU4F2 | 97_Brn3: (POU4F2) _q3F | AACCAGAGGCAGAAACAGAAA | 45 |
| | 98_Brn3: (POU4F2) _q3R | AGGCAGAAGAGAGGAGAGAAA | 46 |
| Onecut1 | 109_Onecut1_q4F | GCTTGGCTGTTTAGTGGTTTG | 47 |
| | 110_Onecut1_q4R | GTGGCATGGTAGAACAGATGAG | 48 |
| CRYAB | 127_CRYAB_q3F | CGCCTCTTTGACCAGTTCTT | 49 |
| | 128_CRYAB_q3R | TAGAAGGGACTCAGGGAAGTAG | 50 |
| MSI1 | 117_Musashi1[MSI1]_q5F | AAGTTTCCCACCAGGATGAC | 51 |
| | 118_Musashi1[MSI1]_q5R | CAACGCTCTGAACCTCTCTTT | 52 |
| CALB2 | 121_CALB2_q5F | CGCAGACGGAAATGGGTATATT | 53 |
| | 122_CALB2_q5R | GACATCATGCCAGAGCCTTT | 54 |
| PRDM1 | 119_Blimp1[PRDM1]_q3F | TGTGGTATTGTCGGGACTTTG | 55 |
| | 120_Blimpl[PRDM1]_q3R | TCAGTGCTCGGTTGCTTTAG | 56 |
| Onecut2 | 113_Onecut2_q4F | GTCCCTCCTACTGTGTGTTATG | 57 |
| | 114_Onecut2_q4R | CCCTACTAATGGGATGGATGAG | 58 |
| BEST | BEST1_q1F | TCATCCCCATTTCCTTCGTG | 59 |
| | BEST1_q1R | TTGCTCGTCCTTGCCTTC | 60 |
| MERTK | MERTK_q1F | TGGCGGTCTTCAGTTGTG | 61 |
| | MERTK_q1R | GGAACCCAGGAGATCAGAATG | 62 |
| MiTF | 41_MiTF_q1F | AGGAAATCTTGGGCTTGATGG | 63 |
| | 42_MiTF_q1R | TGTTGGGAAGGTTGGCTG | 64 |
| POU5F1 | 69 POU5F1 realtime fw1 | AGAACATGTGTAAGCTGCGG | 65 |
| | 70_POU5F1_q1R | GTTGCCTCTCACTCGGTTC | 66 |
| NANOG | 73 NANOG realtime fw1 | GAAATACCTCAGCCTCCAGC | 67 |
| | 74 NANOG realtime rv1 | GCGTCACACCATTGCTATTC | 68 |
| hLGALS3 | 169_hLGALS3_q3F | GACAGTCGGTTTTCCCATTTG | 69 |
| | 170_hLGALS3_q3R | TCCCAGTTTGCTGATTTCATTG | 70 |
| hS100A10 | 157_hS100A10_q2F | GGGATAAAGGCTACTTAACAAAGG | 71 |
| | 158_hS100A10_q2R | CCACTTTGCCATCTCTACACTG | 72 |
| THRB2 | 221_THRB_q1F | CATCAAAACTGTCACCGAAGC | 73 |
| | 222_THRB_q1R | TCCAAGTCAACCTTTCCACC | 74 |
| sox3 | 161_hSOX3_q1F | CGCTGACCCACATCTGAG | 75 |
| | 162_hSOX3_q1R | TCATCGGTACAAGGCAACAG | 76 |
| SSEA1 | 203_SSEA1_q1F | ACCAACTGAGCCAACATGTG | 77 |
| | 204_SSEA1_q1R | GAGTTCTCGAAAAGCCAGGTAG | 78 |
| ISL1 | 243_ISL1_q1CF | CACTGTGGACATTACTCCCTC | 79 |
| | 244_ISL1_q1CR | CCGCATGCCATTCCAAATC | 80 |
| MSX1 | 245_MSX1_q1F | TCAAGCTGCCAGAAGATGC | 81 |
| | 246_MSX1_q1R | TGTGTTTGCGGAGGGTG | 82 |
| BHLHE23 | 165_hBHLHE23_q2F | CCTGCCCTGACAAGTGC | 83 |
| | 166_hBHLHE23_q2R | CAGTCACAGGGGCGATC | 84 |
| ARR3 | 209 ARR3_q1F | CTGGTGTCCTACAAAGTCAGAG | 85 |
| | 210_ARR3_q1R | TCATGAGATGGCTTCGGATG | 86 |
| | 211_ARR3_q3F | CCAGAAAAGGCTCAACATCAAC | 87 |
| | 212_ARR3_q3R | TTCCACCGTGTCCACATG | 88 |
| CRYGA | 235_hCRYGA | CAGCGACTCGGTCCAATC | 89 |
| | 236_hCRYGA | AGACATAAGGCCTCGGTAGT | 90 |

### 4. Medicament for Treatment of Retinal Degenerative Diseases

The present invention also provides a medicament for the treatment of retinal degenerative diseases, which contains a plurality of retinal sheets for transplantation and a transplantation medium (which also functions as a preservation solution for the sheets). In the medicament, at least some of the plurality of retinal sheets for transplantation are excised from a single planar retinal organoid derived from pluripotent stem cells, and all of them have been confirmed by optical coherence tomography (OCT) to have a thickness of 70 µm or more and to be free of foreign structures. Examples of the "transplantation medium" include a diluted viscoelastic substance solution such as 7-fold diluted Viscoat.

Each sheet is a fragment having a size that can be loaded into a cannula, which is a transplantation instrument, preferably a 18G to 24G cannula, for example, a size with a length of 0.5 to 2.0 mm, preferably 0.5 to 1.0 in the short axis direction and 1 mm or more, preferably 1 to 3 mm in the long axis direction, and therefore a plurality of sheets can be loaded into a 24G cannula for transplantation, allowing for transplantation without making a large incision in the retina.

The plurality of retinal sheets for transplantation contained in the medicament of the present invention are characterized by having a high expression of one or more selected from the photoreceptor progenitor cell markers Crx, Recoverin, and Rxr-gamma, and low expression or below the detection limit of one or more selected from the extraretinal tissue cell markers CRYAB, EMX2, s100A10, Zic1, AQP1, and MSX1, preferably one or more selected from CRYAB, Zic1, and EMX2.

The retinal organoid constituting the retinal sheets for transplantation has a ratio of retinal cells (including retinal progenitor cells) to all cells of about 90% or more, preferably about 95% or more, and the cells are forming a layered structure.

As used herein, a "retinal degenerative disease" is a disease in which abnormalities in visual function occur due to damage to the cells constituting the retinal tissue, and include retinal diseases associated with degeneration, damage, or loss of rod cells or cone cells. Examples of retinal degenerative diseases include retinitis pigmentosa, age-related macular degeneration, macular dystrophy, cone-rod dystrophy, rod-cone dystrophy, macular hole, degenerative myopia, and traumatic macular disease.

### 5. Method for Producing Composite Sheet for Retinal Transplantation

Taking advantage of its sheet-like shape, the retinal organoid can also be laminated with retinal pigment epithelium cells to produce a composite sheet. *In vivo,* the retina and retinal pigment epithelial cells are in contact with each other on their parietal surfaces, and do not require strong adhesion. Therefore, a low-viscosity flowable gel, such as x7 Viscoat (mixture of sodium chondroitin sulfate and sodium hyaluronate for ophthalmic surgery), can be used to loosely adhere the retinal sheet for transplantation and the retinal pigment epithelial cell sheet, and obtain a composite sheet that can be used for transplantation.

The present invention also provides a method for producing a composite sheet for retinal transplantation as described above, the method including a step of producing a retinal sheet for transplantation from a retinal organoid derived from pluripotent stem cells, and a step of laminating the retinal sheet for transplantation onto a retinal pigment epithelial cell sheet via a flowable gel to produce the composite sheet for retinal transplantation.

As a flowable gel, a histocompatible flowable gel can be used, including polysaccharides (including derivatives such as their salts and esters) such as hyaluronic acid and chondroitin sulfate that are present *in vivo* as an extracellular matrix between the retina and the retinal pigment epithelial cells. These flowable gels can also be used as the transplantation medium during sheet insertion, as well as components of the preservation solution for the sheets.

### 6. Method for Treating Retinal Degenerative Diseases

The present invention also provides a method for treating retinal degenerative diseases, which includes preparing a plurality of retinal sheets for transplantation from a retinal organoid derived from pluripotent stem cells according to the method described in 1., and transplanting the plurality of retinal sheets for transplantation (for example, by loading them into a 24G cannula) into the retina of a subject in need thereof.

### 7. Automation Program

The method for preparing the retinal sheet for transplantation of the present invention can be at least partially automated.

For example, the program may include: a step of acquiring OCT information of the retinal organoid; a step of inputting the OCT information into a computer; a step of sequentially generating X-Y planar images from the OCT information and identifying the regions free of foreign structures; a step of generating a heat map from the OCT information and selecting a region with a thickness of 70 µm or more, preferably 70 to 175 µm, and particularly preferably 80 to 150 µm; a step of identifying a region that has a thickness of 70 µm or more and is free of foreign structures as a region suitable for transplantation; and a step of setting a grid of a size corresponding to the retinal sheet for transplantation on the region suitable for transplantation such that the effective number of sheets is maximized, and outputting commands to an excision device to excise the retinal sheet for transplantation along the grid. The program may include, as needed, a step of acquiring information from bright-field microscopy and/or phase-contrast microscopy; a step of inputting the information from the bright-field microscopy and/or phase-contrast microscopy; a step of identifying a region estimated to be a region suitable for transplantation (estimated region) based on the information from the bright-field microscopy and/or phase-contrast microscopy; and a step of issuing a command to perform OCT on the estimated region.

The above is an example of the steps of the program, and the automation program of the present invention may include some of these steps, or may include additional steps.

### Examples

Hereinafter, the present invention will be described more specifically with Reference Examples and Examples. However, the present invention is not limited to these Reference Examples and Examples.

### Reference Example 1: Production of retinal organoid from pluripotent stem cells

A planar retinal organoid was produced according to the following method.

### Day 0

A PDMS sheet having a hole with a diameter of 1 cm is placed on a 35 mm dish, and the dish was coated in a circular manner such that the inside of the hole is filled with 200 µl of 7 µl iMatrix-511/500 µl PBS dilution (at 37°C for 1 hour). After removing the coating agent with an aspirator, 5×10⁵ iPS cells are suspended in 200 µL of StemFIT^{®} AK02N medium supplemented with Y27632 at a final concentration of 10 µM, and seeded onto the same area. After allowing the dish to stand in a 37°C incubator for 30 minutes, the PDM sheet is removed using sterile tweezers, and 300 µL of the above StemFIT^{®} AK02N+Y27632 medium is added, followed by culture in a 5% CO₂ incubator.

### Day 1

The medium is replaced with 500 µL of StemFIT AK02N medium (without Y27632 and supplemented with penicillin/streptomycin).

### Day 2

After washing three times with 1 mL of gfCDM (10% KSR), the medium is completely replaced with 1 mL of gfCDM (10% KSR).

### [Composition of gfCDM medium]

| | |
|---|---|
| IMDM (31980-030) | 125 ml |
| F-12 (31765-092) | 125 ml |
| CD lipid (11950-031) | 2.5 ml |
| BSA (5g/20ml) | 5 ml |
| 1-Thioglycerol (207-09232) | 9.75 µl |
| Penicillin/streptomycin | 2.5 mL |
| KSR | 25 ml |

### Day 3 and Day 6

The medium is replaced with 1 mL of gfCDM medium containing 100 nM LDN.

### Day 8

After washing with 1 mL of gfCDM, the medium is replaced with 1 mL of medium containing BMP4 (final concentration of 1.5 nM).

### Day 11 and Day 14

Half of the gfCDM medium is replaced.

### Day 17

After washing three times with DMEM/F-12 + GlutaMAX, the medium is replaced with 1 mL of the medium described below.

### [Medium Composition]

| | |
|---|---|
| DMEM/F-12+Glutamax (10565-042) | 5 ml |
| N2 supplement (17502-048) | 50 µl |
| Penicillin/streptomycin | 50 µl |

### Day 20

The medium is replaced with 1 mL of the following control mature medium.

### [Medium Composition]

| | |
|---|---|
| DMEM/F-12+Glutamax (10565-042) | 500 ml |
| N2 supplement (17502-048) | 5 ml |
| Inactivated FBS | 50 mL |
| 50 mM Taurine (in PBS) | 1.12 ml |
| Antibiotic-Antimycotic (100x) (15240-062) | 5 ml |

| | |
|---|---|
| *Thereafter, the medium is replaced once every 2 to 3 days. | |

### Example 1: Observation of retinal organoid

In a retinal planar organoid at around 60 days of differentiation, the thickness of the retinal organoid and the regions free of foreign structures were identified using 1) bright-field microscopy (assessment of the overall condition), 2) phase-contrast microscopy (observation of homogeneity), 3) OCT cross-sectional imaging (information on uniformity, thickness, and foreign structures), and 4) OCT X-Y planar observation.

### 1. Bright-field Microscopy

First, the overall condition was assessed by bright-field microscopy. When differentiation into the retina is observed, the organoid exhibits, in all cases, the formation of characteristic RPE or three-dimensional organoid-like structures at the periphery, and these structures serve as corroborative evidence of retinal differentiation. In addition, the thick areas can be observed as light brown, and the overall uniformity can be roughly observed by bright-field microscopy (Figure 1).

### 2. Phase-contrast Microscopy

When phase-contrast microscopy was performed on the organoid, the internal structure could be observed in greater detail, and the areas within the sheet exhibiting uniform structure could be identified (Figure 2).

### 3. Optical Coherence Tomography (OCT)

Cross-sectional images were obtained using optical coherence tomography (OCT) by Screen. Differences in brightness allowed for the detection of foreign structures within the tissue and confirmation of uniformity (Figure 3), indicating that examining the brightness distribution allows identification of the presence of foreign matter. Furthermore, by sequentially extracting a plurality of horizontal X-Y planar images from the OCT cross-sectional images along the Z direction at intervals of 10 µm or less, and sequentially evaluating the X-Y horizontal planes, it is possible to more efficiently determine the absence of foreign structures with differing brightness distributions (Figure 4).

The thickness information from the OCT cross-sectional images was exported as a heat map (Figure 5). This allows identification of the overall thickness trend by utilizing the heat map after confirmation of the internal structure by OCT cross-sectional images. Furthermore, by converting the heat map into a binarized image, it is possible to identify graft areas having an appropriate thickness, such as regions with a thickness of 80 µm or more, or 75 to 125 µm.

### 4. Quantitative PCR

After confirming the estimated retinal region, a graft of 1 x 2 mm in size was excised from within the region, and its gene expression pattern was confirmed using a PCR panel. This allows assessment of contamination by extraretinal tissue, the determination of the general composition of inner cells/photoreceptor cells within the retinal cells at 60 days of differentiation, and the like. As a result, it was confirmed that ciliary body/lens markers were high in a thin retina (Figure 6). On the other hand, the expression levels of marker genes for extraretinal tissues were low in the thick and uniform regions (4, 5, 9, and 10), confirming that these areas were suitable for transplantation.

The quantitative PCR results were compared between sheets having the same retinal gene profile using a radar chart. A decrease in the expression of progenitor cell markers and an increase in the expression of photoreceptor cell markers from days 40 to 60 of differentiation can be observed, which indicates that the expression of these gene groups changes relative to each other, depending on the number of days of differentiation (Figure 11A). The retinal gene expression patterns at days 50 and 60 of differentiation can be evaluated and determined to be nearly identical (Figure 11B). By feeding back the results post-transplantation and collecting the qPCR data of transplantation sheets that showed good engraftment (Figure 11C), a range of retinal gene expression patterns indicating a good sheet (gray band in Figure 11C) can be identified, and the sheets exhibiting gene expression patterns that deviate from this pattern can be considered unsuitable.

### 5. Preparation of Graft

The above results confirm that the region suitable for transplantation of the retinal organoid can be identified as a region with a uniform thickness (70 µm or more) and free of foreign structures with differing brightness distributions. The thickness and the presence or absence of foreign structures can be determined by producing sequential X-Y planar images and heat maps from OCT cross-sectional images. By narrowing down the regions to be evaluated by OCT using bright-field microscopy or phase-contrast microscopy, it is possible to identify more efficiently the regions suitable for transplantation.

For example, for a planar organoid sheet with a diameter of about 1 cm, the regions with uniformity that are suitable for transplantation are estimated using bright-field/phase-contrast microscopy, and then, using OCT cross-sectional images, transplant tissues of 1 x 2 mm are sequentially excised from the region with a thickness of about 70 µm or more and with uniformity. As described above, the evaluation of thickness can be efficiently conducted by using a heat map. For the excision of the graft, a grid corresponding to the size of the graft is set on the retinal organoid, and alignment is performed such that the effective number of sheets is maximized on the region suitable for transplantation, then the grafts are excised along the grid (Figure 8). A plurality of 1 x 2 mm grafts can be loaded into a 24G cannula for transplantation, thus allowing transplantation without making a large incision in the retina (Figure 9).

In addition, by superposing a separately prepared sheet of retinal pigment epithelial cells onto the planar organoid sheet with their parietal sides in contact with each other, a composite sheet that mimics the *in vivo* relationship between the two can be prepared for transplantation. While the retina and retinal pigment epithelial cells are originally in functional contact with each other on the parietal side, there is no connective tissue between them during development. If the two can be temporarily held together as a composite at the time of transplantation, it is believed that each will engraft on its respective defined surface post-transplantation, thereby establishing a functional relationship thereafter.

Therefore, as shown in Figure 10, a viscoelastic medium (35,000 to 60,000 mPa·s (25°C, shear rate of 2 s⁻¹), diluted 2-fold) was placed on the retinal organoid, and a retinal pigment epithelial cell sheet was further layered on top such that its parietal side was in contact with the organoid. The sheets may either be cut to an appropriate size before being layered, or layered then cut to the desired size. After the superposition, any excess viscoelastic substance is removed as much as possible using MQA^{®} or the like. The resulting composite sheet can be inserted into a 24G cannula together with a transplantation medium of similar or lower viscosity, and it can also be extruded from the cannula while maintaining a composite sheet state.

### Industrial Applicability

According to the method of the present invention, a sheet suitable for transplantation can be efficiently excised from a retinal organoid by an objective method of evaluation, without relying on the special technique of an expert. The present invention makes a significant contribution to the practical application of regenerative medicine for retinal degenerative diseases.

All prior art documents cited in the present specification are incorporated herein by reference.

## Claims

1. A method for producing a retinal sheet for transplantation from a retinal organoid derived from pluripotent stem cells, the method comprising: a step of performing one or more selected from optical coherence tomography (OCT), confocal image analysis, and acoustic microscopy analysis on the retinal organoid derived from pluripotent stem cells to identify, as a region suitable for transplantation, a region with a thickness of 70 µm or more and free of foreign structures **characterized by** differences in brightness, and a step of excising a fragment from the region suitable for transplantation to obtain the retinal sheet for transplantation.

2. The method according to claim 1, wherein the region free of foreign structures is identified by sequentially extracting a plurality of horizontal X-Y planar images reconstructed from OCT cross-sectional images, along the Z direction, and evaluating them.

3. The method according to claim 1, wherein the region with a thickness of 70 µm or more is identified using a heat map.

4. The method according to claim 1, wherein the excised fragment is of a size that can be loaded into an 18G to 24G cannula.

5. The method according to claim 1, wherein the excised fragment has a length of 1.0 to 10.0 mm in the long axis direction and a length of 0.5 to 2.0 mm in the short axis direction.

6. The method according to claim 1, further comprising a step of performing bright-field microscopy and/or phase-contrast microscopy on the retinal organoid derived from pluripotent stem cells,
wherein the identification of the region suitable for transplantation by the OCT is performed on a region where retinal differentiation and homogeneity can be confirmed by the bright-field microscopy and/or phase-contrast microscopy.

7. The method according to claim 1, wherein the retinal organoid is a planar retinal organoid with a diameter of 1 cm or more.

8. A method for producing a composite sheet for retinal transplantation, the method comprising: a step of producing a retinal sheet for transplantation by the method according to any one of claims 1 to 7; and a step of laminating the retinal sheet for transplantation onto a retinal pigment epithelial cell sheet via a flowable gel to produce the composite sheet for retinal transplantation.

9. A medicament for the treatment of retinal degenerative diseases, comprising a plurality of cell sheets for retinal transplantation excised from a single planar retinal organoid derived from pluripotent stem cells, and a transplantation medium, wherein the plurality of cell sheets for retinal transplantation each have a size with a length of 1.0 to 10.0 mm in the long axis direction and 0.5 to 2.0 mm in the short axis direction, and have been confirmed by optical coherence tomography (OCT) to have a thickness of 70 µm or more and to be free of foreign structures.
